# EUROPÄISCHE PATENTSCHRIFT

(11) **EP 3 010 597 B1**
(45) Veröffentlichungstag und Bekanntmachung des Hinweises auf die Patenterteilung: **05.07.2017**
(21) Anmeldenummer: 14701761.0
(22) Anmeldetag: 29.01.2014
(51) Int. Cl.: A61K 8/25, A61Q 11/00, A61Q 11/02, A61K 8/73, A61K 8/81

(54) **MUND- UND ZAHNPFLEGE- UND -REINIGUNGSMITTEL ZUR VERRINGERUNG DER WIEDERANFÄRBUNG VON ZÄHNEN**
ORAL AND DENTAL HYGIENE- AND CLEANING AGENTS FOR REDUCING RE-STAINING OF TEETH
PRODUITS DE NETTOYAGE ET D'HYGIÈNE BUCCODENTAIRE DESTINÉS À LIMITER LA RECOLORATION DES DENTS

(30) Priorität: 19.06.2013 DE 102013211519
(43) Veröffentlichungstag der Anmeldung: 27.04.2016
(73) Patentinhaber: Henkel AG & Co. KGaA, 40589 Düsseldorf (DE)
(72) Erfinder: MIEHLICH, Kristin, 42119 Wuppertal (DE); WELSS, Thomas, 40591 Düsseldorf (DE); ARIANS, Daniela, 45239 Essen (DE); BIDAULT, Samantha, F-78830 Bullion (FR)
(86) Internationale Anmeldenummer: PCT/EP2014/051682
(87) Internationale Veröffentlichungsnummer: WO 2014/202232

(56) Entgegenhaltungen:
- EP-A2- 2 556 817
- WO-A1-03/042251
- DE-A1-102010 062 611
- HIROSHI SANO ET AL: "Effect of chitosan rinsing on reduction of dental plaque formation.", BULLETIN OF TOKYO DENTAL COLLEGE, Bd. 44, Nr. 1, 1. Februar 2003 (2003-02-01), Seiten 9-16, XP055015488, ISSN: 0040-8891
- GANSS C ET AL: "Toothpaste and Erosion", 1. Januar 2013 (2013-01-01), TOOTHPASTES, KARGER MEDICAL AND SCIENTIFIC PUBLISHERS, PAGE(S) 88 - 99, XP009177755, ISBN: 978-3-318-02206-3 Seite 95, linke Spalte, Absatz 1-2
- LYNCH RJ: "Calcium glycerophosphate and caries: a review of the literature", INTERNATIONAL DENTAL JOURNAL, BUTTERWORTH AND CO., LTD, GB, Bd. 54, Nr. 5, Suppl.1, 1. Januar 2004 (2004-01-01), Seiten 310-314, XP009177764, ISSN: 0020-6539

## Beschreibung

Die Erfindung betrifft Mund- und Zahnpflegemittel mit einer Wirkstoffkombination zur schonenden und effektiven Reinigung der Zähne.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Viele Menschen wünschen sich weiße Zähne und empfinden dunkle oder verfärbte Zähne als kosmetisch inakzeptabel. Die Aufrechterhaltung der natürlichen Zahnfarbe ist jedoch trotz regelmäßiger Dentalhygiene nicht immer erfolgreich. Ernährungsgewohnheiten oder Rauchen können zu Zahnverfärbungen führen. Ebenso führt die Besiedlung der Zahnoberfläche mit Bakterien (Plaque) zu Verfärbungen.
Eine Reihe von technischen Lösungen zur Entfernung oder Aufhellung von Zähnen wurde entwickelt. Zur Aufhellung/Bleichen kommt vor allem Peroxid zum Einsatz. In professionellen Bleichprodukten wird Peroxid in hohen Konzentrationen eingesetzt, während der Einsatz in Kosmetikprodukten zur Mund- und Zahnpflege auf 0,1% Peroxid beschränkt ist. Peroxid in dieser Konzentration hat nur eine eingeschränkte aufhellende Wirkung und kann Verfärbungen der Zähne oft nicht im gewünschten Maß beseitigen.
Eine weitere Möglichkeit zur Aufhellung der Zähne besteht in der effektiven Entfernung von Zahnbelägen, welche die Zähne dunkler aussehen lassen. Diese Methode der Zahnaufhellung wird auch als "Natural Whitening" beschrieben. Eine hohe Reinigungsleistung wird am besten erreicht durch Putzkörper, zum Beispiel Silica, Alumina oder Calciumcarbonat in Kombination mit einem Tensid. Leider weisen Zahncremes mit einem effektiven System aus einem oder mehreren Putzkörpern häufig auch eine hohe Abrasivität auf, führen also zu einem gewissen, wenn auch sehr geringem Abrieb der Zahnoberfläche. Dies kann sich insbesondere dann nachteilig auswirken, wenn das Enamel eines Zahnes ohnehin dünn ist, wie dies bei Personen mit empfindlichen Zähnen der Fall ist. Oft treten bei Personen mit empfindlichen Zähnen auch freiliegende Zahnhälse auf, also Partien des Zahnes in unmittelbarer Nähe zum Zahnfleisch, an denen kein Enamel als Schutzschicht vorhanden ist und das darunter liegende Dentin frei liegt.

Zudem bildet sich sofort nach dem Zähneputzen auf den Zahnmaterial eine Eiweißschicht (Pellicle) auf der sich der Plaque aufbaut bzw. Verfärbungen anlagern.

Es besteht daher ein Bedarf an Zahnpasten, die eine effektive Reinigung und Aufhellung bewirken aber dabei gleichzeitig einen Langzeiteffekt bewirken, d.h. die Bildung neuer Plaque und damit neuer Verfärbungen möglichst lange verhindern bzw. verringern.

Die Aufgabe der vorliegenden Erfindung bestand darin, Zubereitungen zur Mund- und Zahnpflege und -reinigung bereitzustellen, die eine effektive Reinigung und Aufhellung bewirken und dabei neue Verfärbungen möglichst lange verhindern bzw. verringern.

Die DE 10 2010 062611 A1 offenbart antibakterielle Zubereitungen zur Mund- und Zahnpflege und -reinigung, enthaltend 4-(3-Ethenyl-3,7-dimethyl-1,6-octadienyl)-phenol als antibakterielles Mittel. Laut Beschreibung können sowie Chitosanderivate, PVM/MA-Copolymere und Fällungskieselsäuren in den Mitteln enthalten sein.

Die EP 2 556 817 A2 beschreibt Mund- und Zahnpflege und -reinigungsmittel, die 1 bis 30 Gew.-% Fällungskieselsäuren mit spezifischen Oberflächen nach ISO 5794-I, Anhang D von ≤ 55 m²/g enthalten. Die Zusammensetzungen können ferner noch Ca-Glycerophosphat enthalten und dienen der Aufhellung von Zähnen bzw. der Beseitigung von Zahnverfärbungen.

In der WO 03/042251 A1 werden chitosan- und kieselsäurehaltige Mund- und Zahnpflege und -reinigungsmittel beschreiben. Die Zusammensetzungen können ferner noch PVM/MA-Copolymere und Ca-Glycerophosphat enthalten.

In der XP055015488 wird offenbart, daß Chitosan die Plaquebildung reduziert.

Die XP009177755 beschreibt Chitosan in der Zahnpflege sowie dessen antibakterielle und gewebsregenierungsanregende Wirkung.

In der XP009177764 wird die Anti-Karies-Wirkung von Ca-Glycerophosphat offenbart.

Überraschenderweise wurde nun gefunden, dass eine Kombination zweier Polymere mit bestimmten Abrasivstoffen dazu führt, dass Anfärbungen (z.B. aus der Nahrung, insbesondere Teeanfärbungen) sichtbar weniger aufgenommen werden. Die schonende Reinigung wird durch eine solche Kombination um eine Langzeitwirkung gegen Wiederanschmutzung ergänzt.

Gegenstand der Erfindung sind daher Mund- und Zahnpflege- und -reinigungsmittel, enthaltend - bezogen auf sein Gewicht -
a) 0,001 bis 5 Gew.-% Copolymer(e), umfassend mindestens eine Struktureinheit der Formel (A1) und mindestens eine Struktureinheit der Formel (A2) 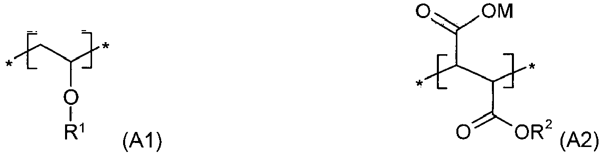 worin
   - R¹: steht für eine (C₁ bis C₁₈)-Alkylgruppe,
   - R²: steht für eine (C₁ bis C₆)-Alkylgruppe,
   - M: steht für ein Wasserstoffatom oder ein Äquivalent eines ein- oder mehrwertigen Kations,
b) 0,001 bis 5 Gew.-% Polymer(e) aus der Gruppe Chitosan und Chitosanderivate;
c) 1 bis 20 Gew.-% Fällungskieselsäure(n) mit einer spezifischen Oberfläche nach ISO 5794-1, Anhang D von ≤ 60 m²/g.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Zusammensetzungen bezogen auf ihr Gewicht 0,001 bis 5 Gew.-% Copolymer(e), umfassend mindestens eine Struktureinheit der Formel (A1) und mindestens eine Struktureinheit der Formel (A2) 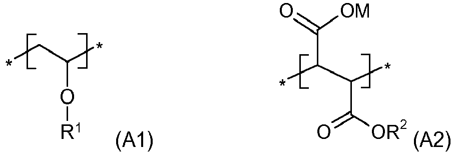 worin
- R¹: steht für eine (C₁ bis C₁₈)-Alkylgruppe,
- R²: steht für eine (C₁ bis C₆)-Alkylgruppe,
- M: steht für ein Wasserstoffatom oder ein Äquivalent eines ein- oder mehrwertigen Kations.

Gemäß obiger Formeln und allen folgenden Formeln steht eine chemische Bindung, die mit dem Symbol * gekennzeichnet ist, für eine freie Valenz des entsprechenden Strukturfragments.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.
Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Es ist erfindungsgemäß bevorzugt, wenn die erfindungsgemäßen Mittel als Komponete (b) mindestens ein Polymer enthalten, ausgewählt aus mindestens einem Polymer der Gruppe mit Polymeren der INCI-Nomenklatur Butyl Ester of PVM/MA Copolymer, Isopropyl Ester of PVM/MA Copolymer, Ethyl Ester of PVM/MA Copolymer.

Entsprechende Polymere der Komponete a) des erfindungsgemäßen Mittels werden beispielsweise unter dem Handelsnamen Gantrez ES 425 (Copolymer von Methylvinylether und dem Butyl-Halbester der Maleinsäure; 50 Gew.-% Aktivsubstanz in Ethanol; INCI-Bezeichnung: Butyl Ester of PVM/MA-Copolymer (Ashland)), Gantrez^{®} ES 435 (Copolymer von Methylvinylether und dem Butyl-Halbester der Maleinsäure; 50 Gew.-% Aktivsubstanz in Isopropanol; INCI-Bezeichnung: Butyl Ester of PVM/MA-Copolymer (Ashland)), Gantrez^{®} ES 335I (Copolymer von Methylvinylether und dem Isopropyl-Halbester der Maleinsäure; 50 Gew.-% Aktivsubstanz in Isopropanol; INCI-Bezeichnung: Butyl Ester of PVM/MA-Copolymer (Ashland)), Gantrez^{®} ES 225 (Copolymer von Methylvinylether und dem Ethyl-Halbester der Maleinsäure; 50 Gew.-% Aktivsubstanz in Ethanol) INCI-Bezeichnung: Ethyl Ester of PVM/MA-Copolymer (Ashland)).

Ganz besonders bevorzugte Polymere a) weisen mindestens eine Struktureinheit der Formel (A1) und mindestens eine Struktureinheit der Formel (A2) auf, in denen R¹ = R² = M = -H gilt, d.h. Copolymere von Methylvinylether und maleinsäure sind erfindungsgemäß besonders bevorzugt einsetzbar. Solche Copolymere beispielsweise unter dem Handelsnamen Gantrez^{®} S 97 BF (Copolymer von Methylvinylether und Maleinsäure (Ashland)) vertrieben.

Besonders bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Copolymer(e) aus Maleinsäure und Methylvinylether enthalten.

Es hat sich gezeigt, dass Copmere a) eines bestimmten Mnolmassenbereiches in der erfindungsgemäßen Kombination besonders wirksam sind. Besonders bevorzugte erfindungsgemäße Mund- und Zahnpflege- und reinigungsmittel sind daher dadurch gekennzeichnet, dass das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

Ganz besonders bevorzugt ist wiederum der Einsatz von Copolymeren aus Methylvinylether und Maleinsäure, so dass insbesondere bevorzugte Mund- und Zahnpflege- und reinigungsmittel dadurch gekennzeichnet sind, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Copolymer(e) aus Methylvinylether und Maleinsäure enthalten, das/die Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

Als zweiten wesentlichen Inhaltsstoff enthalten die erfindungsgemäßen Mittel 0,001 bis 5 Gew.-% Polymer(e) aus der Gruppe Chitosan und/oder mindestens eines seiner Derivat(e).

Chitosan ist das Deacetylierungsprodukt von Chitin, wobei ein definierter Übergang zwischen Chitosan und Chitin nicht existiert. Im Rahmen der vorliegenden Erfindung wird von Chitosan gesprochen, wenn der Deacetylierungsgrad des Chitins mindestens 20 % beträgt. Bevorzugte erfindungsgemäße Mittel enthalten Chitosane (bzw. Derivate davon), deren Deacetylierungsgrad > 30 %, insbesondere >40-50% beträgt. Weiter bevorzugt sind Chitosane und Derivate davon, die in organischen Säuren löslich sind.

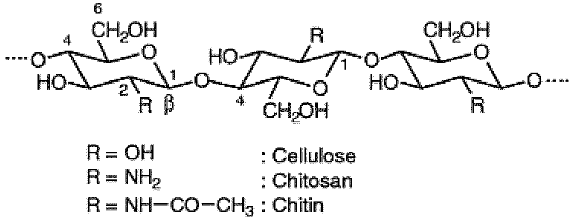

Bevorzugte erfindungsgemäß eingesetzte Chitosane oder Derivate davon weisen Molmassen von 100.000 bis 500.000 Dalton auf. Chitosan besteht aus Ketten von beta-1,4-glycosidisch verknüpften N-Acetyl-D-glucosamin-(NAG-)Resten.

Bevorzugte erfindungsgemäße Mund- und Zahnpflege- und -reinigungsmittel sind dadurch gekennzeichnet, dass sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Polymer(e) aus der Gruppe Chitosan und Chitosanderivate enthalten.

Vorzugsweise sind die in den erfindungsgemäßen Mitteln eingesetzten Chitosane bzw. deren Derivate wasserlöslich. Bevorzugte erfindungsgemäße Mittel sind dadurch gekennzeichnet, dass sie Chitosanderivat(e) enthalten, die eine Wasserlöslichkeit in destilliertem Wasser oberhalb von 1 g/l (20°C, 1013 mbar) aufweisen.

Bevorzugt einzusetzende Chitosanderivate sind an der Aminogruppe derivatisiert. Besonders bevorzugte Chitosanderivate weisen einen an das Stickstoffatom der Aminogruppe gebundenen Säurerest, vorzugsweise den Rest einer organischen Säure, auf. Besonders bevorzugte Chitoanderivate weisen mindestens einen an das Stickstoffatom der Aminogruppe gebundenen Säurerest folgender Säuren auf: Ameisensäure, Essigsäure, Propionsäure, Buttersäure, Valeriansäure, Capronsäure, Heptansäure, Caprylsäure, Pelargonsäure, Caprinsäure, Undecansäure, Laurinsäure, Tridecansäure, Tetradecansäure, Pentadecansäure, Palmitinsäure, Margarinsäure, Stearinsäure, Nonadecansäure, Arachinsäure, Behensäure, Lignocerinsäure, Cerotinsäure, Melissinsäure, Isobuttersäure, Isovaleriansäure, Tubercolostearinsäure, Acrylsäure, Crotonsäure, Palmitoleinsäure, Ölsäure, Erucasäure, Sorbinsäure, Linolsäure, Linolensäure, Elaeostearinsäure, Arachidonsäure, Clupanodonsäure, Docosahexaensäure, Oxalsäure, Malonsäure, Bernsteinsäure, Glutarsäure, Adipinsäure, Pimelinsäure, Azelainsäure, Sebacinsäure, Maleinsäure, Fumarsäure, Muconsäure, Phthalsäure, Terephthalsäure.

Besonders bevorzugte erfindungsgemäße Mittel sind dadurch gekennzeichnet, dass das/die in ihnen enthaltene(n) Chitosanderivat(e) ausgewählt ist/sind aus
- Chitosanglycolat
- N-Acetyl-Chitosan (Chitosanacetat)
- N-Propionyl-Chitosan (Chitosanpropionat)
- N-Butanoyl-Chitosan (Chitosanbutanoat)
- N-Malonyl-Chitosan (Chitosanmalonat)
- N-Succinyl-Chitosan (Chitosansuccinat)
- N-Adipyl-Chitosan (Chitosanadipat).

Als dritten wesentlichen Inhaltsstoff enthalten die erfindungsgemäßen Zusammensetzungen bezogen auf ihr Gewicht 1 bis 20 Gew.-% Fällungskieselsäure(n) mit einer spezifischen Oberfläche nach ISO 5794-1, Anhang D von ≤ 60 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskiselsäuren eingesetzt, die noch niedrigere spezifische Oberflächen nach ISO 5794-1, Anhang D aufweisen. Bevorzugte erfindungsgemäße Mund- und Zahnpflege- und - reinigungsmittel enthalten 2,5 bis 19,5 Gew.-%, vorzugsweise 5 bis 19 Gew.-%, besonders bevorzugt 7,5 bis 18,5 Gew.-%, weiter bevorzugt 8,0 bis 18 Gew.-% und insbesondere 10,0 bis 17,5 Gew.-% Fällungskieselsäure(n) mit einer spezifischen Oberfläche nach ISO 5794-1, Anhang D von ≤ 55 m²/g.

Besonders bevorzugte erfindungsgemäße Mund- und und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 60 m²/g, vorzugsweise von ≤ 52,5 m²/g, weiter bevorzugt von ≤ 49 m²/g und insbesondere von ≤ 47 m²/g aufweisen.
In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Die erfindungsgemäßen Mittel können zusätzlich zu den genannten Fällungskieselsäuren a) weitere Poliermittel enthalten. Als Poliermittel eignen sich prinzipiell alle für Zahnpasten bekannten Reibkörper, insbesondere solche, die keine Calciumionen enthalten. Bevorzugt geeignete Poliermittelkomponenten sind daher Aluminiumhydroxid, Aluminiumoxid, Natrium-aluminiumsilikate, organische Polymere oder Gemische solcher Reibkörper.

Es ist bevorzugt, dass die erfindungsgemäßen Zusammensetzungen nur wenig bis keine Fällungskieselsäuren enthalten, die eine spezifische Oberfläche nach ISO 5794-1, Anhang D von > 55 m²/g aufweisen. Sollen solche Kieselsäuren eingesetzt werden, liegt das Gewichtsverhältnis von Fällungskieselsäuren mit einer spezifischen Oberfläche nach ISO 5794-1, Anhang D von ≤ 55 m²/g (Inhaltsstoff c)) zu Fällungskieselsäuren mit einer spezifischen Oberfläche nach ISO 5794-1, Anhang D von > 55 m²/g vorzugsweise > 1:1, weiter bevorzugt > 2:1, noch weiter bevorzugt > 5:1, besonders bevorzugt > 10:1 und insbesondere > 50:1.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Als weiteren Inhaltsstoff können die erfindungsgemäßen Mittel 0,001 bis 10,0 Gew.-% mindestens eines Calciumsalzes enthalten. Besonders bevorzugt werden Calciumsalz(e) innerhalb engerer Mengenbereiche eingesetzt, so dass bevorzugte Mund und Zahnpflege- und -reinigungsmittel 0,05 bis 7,5 Gew.-%, vorzugsweise 0,1 bis 5 Gew.-%, weiter bevorzugt 0,15 bis 2,5 Gew.-% und insbesondere 0,2 bis 1,25 Gew.-% Calciumsalz(e) enthalten.

Es lassen sich erfindungsgemäß alle physiologisch verträglichen Calciumsalze einsetzen, bevorzugt ist der Einsatz von Calciumsalzen, die einen weiteren Nutzen im erfindungsgemäßen Mund und Zahnpflege- und -reinigungsmittel entfalten. Unter diesen Verbindungen ganz besonders bevorzugt sind Calciumhydrogenphosphat-Dihydratn und/oder Calcium-Glycerophosphat.

Calciumhydrogenphosphat-Dihydrat, CaHPO₄•2H₂O wird je nach Literaturstelle auch als "Bruschit" oder als Dicalciumphosphat-dihydrat bezeichnet. Erfindungsgemäß bevorzugt ist der Einsatz von CaHPO₄•2H₂O, das durch die CAS-Nr: 7789-77-7 beschrieben wird.

Erfindungsgemäße Mund und Zahnpflege- und -reinigungsmittel, die als Calciumsalz Calciumhydrogenphosphat-Dihydrat, CaHPO₄-2H₂O enthalten, weisen deutliche Abrasivitätsvorteile gegenüber anderen Mitteln auf, die schonende Reinigung sensitiver Zähne ist daher mit der erfindungsgemäßen Kombination von Polymilchsäure, definiertem Silikat und Calciumhydrogenphosphat-Dihydrat als Calciumsalz noch einmal besser.

Es hat sich gezeigt, dass Calciumhydrogenphosphat-Dihydrat, CaHPO₄•2H₂O, vorzugsweise innerhalb enger Mengenbereiche eingesetzt wird. Hier sind erfindungsgemäße Mund und Zahnpflege- und - reinigungsmittel bevorzugt, die 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat enthalten.

Zusätzlich zu Calciumhydrogenphosphat-Dihydrat oder an seiner Stelle können die erfindungsgemäßen Mittel mit besonderem Vorzug Calcium-Glycerophosphat, d.h. ein Calciumsalz mindestens einer Glycerophosphorsäure enthalten.

Die Glycerophosphorsäure ist eine zweibasige Säure, die in zwei isomeren Formen vorkommt, je nachdem, ob die Phosphorsäuregruppierung an eine terminale oder die mediale OH-Gruppe des Glycerins gebunden vorliegt. Die Form, bei der die Phosphorsäuregruppierung an eine terminale OH-Gruppe des Glycerins gebunden vorliegt, wird auch als alpha-Isomer, die Form, bei der die Phosphorsäuregruppierung an die mediale OH-Gruppe des Glycerins gebunden vorliegt, auch als beta-Isomer bezeichnet.

Das alpha-Isomer ist zusätzlich optisch aktiv und tritt in den zwei enantiomeren Formen sn-Glycerol-1-phosphorsäure 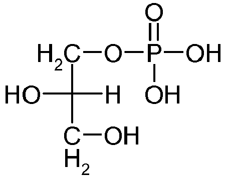 sowie sn-Glycerol-3-phosphorsäure 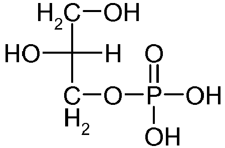 auf.
Das Präfix *sn* bei Glycerol-Derivaten steht für "stereospezifisch nummeriert" und verlangt, dass die 2-Hydroxy-Gruppe in der vorstehend verwendeten Fischer-Projektion nach links weist. Glycerol-2-phosphat ist nicht optisch aktiv. Die Glycerophosphorsäuren sind etwa so stark wie Phosphorsäure.

Erfindungsgemäß bevorzugt ist der Einsatz des alpha-Isomers, unabhängig davon, welches der beiden Enantiomere eingesetzt wird. Sofern der Einsatz enantiomerenreiner Verbindungen gewünscht ist, wird vorzugsweise das Calciumsalz der sn-Glycerol-3-phosphorsäure eingesetzt.

Zusammenfassend sind erfindungsgemäße Mund und Zahnpflege- und -reinigungsmittel bevorzugt, die Calciumsalze der Glycerophosphorsäuren der Formeln (la) und (Ib) enthalten

HO-CH₂-CH(OH)-CH₂-OP(O)O₂²⁻ Ca²⁺ (Ia)

HO-CH₂-CH(OP(O)O₂²⁻)-CH₂-OH Ca²⁺ (Ib).

Hierbei sind besonders bevorzugte erfindungsgemäße Mund und Zahnpflege- und reinigungsmittel dadurch gekennzeichnet, dass das Gewichtsverhältnis der Calciumsalze der Formeln (la) zu (Ib) oberhalb von 50:50, vorzugsweise oberhalb von 60:40, besonders bevorzugt oberhalb von 70:30 und insbesondere oberhalb 80:20 liegt.

Der Einsatz der Calcium-Glycerophosphate innerhalb engerer Mengenbereiche ist bevorzugt. Bevorzugte erfindungsgemäße Mund- und Zahnpflege- und -reinigungsmittel enthalten - bezogen auf ihr Gewicht - 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Ganz besonders bevorzugte erfindungsgemäße Mittel enthalten 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat und 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.
Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und - diglyceriden, von Fettsäure-Sorbitanestern und Alkyl(oligo)-Glucoside sowie Fettsäureamidoalkylbetaine.

Es ist erfindungsgemäß bevorzugt, den Einsatz von Tensiden weitestgehend zu beschränken, um die desensibilisierende Wirkung der erfindungsgemäßen Kombination noch deutlicher in den Vordergrund treten lassen zu können. Daher sind erfindungsgemäße Mund und Zahnpflege- und -reinigungsmittel, die - bezogen auf ihr Gewicht - weniger als 5 Gew.-%, vorzugsweise weniger als 4 Gew.-%, besonders bevorzugt weniger als 3 Gew.-% und insbesondere weniger als 2 Gew.-% Tensid(e) enthalten, erfindungsgemäß besonders bevorzugt.

Ganz besonders bevorzugt ist es, insbesondere den Einsatz anionischer Tenside weitestgehend zu beschränken oder ganz auf diese Tenside zu verzichten. Hier sind bevorzugte erfindungsgemäße Mund und Zahnpflege- und -reinigungsmittel dadurch gekennzeichnet, dass sie weniger als 2 Gew.-%, vorzugsweise weniger als 1 Gew.-%, besonders bevorzugt weniger als 0,5 Gew.-% und insbesondere weniger als 0,1 Gew.-% anionische(s) Tensid(e) enthalten, wobei bevorzugte Mittel frei von anionischen Tensiden sind.

Sofern Tenside - vorzugsweise innerhalb der vorstehend genannten Höchstgrenzen - eingesetzt werden sollen, ist der Einsatz amphoterer Tenside bevorzugt. Bevorzugte tensidhaltige erfindungsgemäße Mund und Zahnpflege- und -reinigungsmittel enthalten 0,1 bis 5 Gew.-%, vorzugsweise 0,25 bis 4 Gew.-%, besonders bevorzugt 0,5 bis 3,0 Gew.-%, weiter bevorzugt 0,75 bis 2,0 Gew.-% und insbesondere 1,0 bis 1,5 Gew.-% amphotere(s) Tensid(e).

Insbesondere bevorzugte erfindungsgemäße Mund und Zahnpflege- und -reinigungsmittel dieser Ausführungsform enthalten 0,1 bis 5 Gew.-%, vorzugsweise 0,2 bis 4 Gew.-%, besonders bevorzugt 0,25 bis 3 Gew.-%, weiter bevorzugt 0,3 bis 2 Gew.-% und insbesondere 0,4 bis 0,8 Gew.-% Cocoamidopropylbetain.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.
Hier sind erfindungsgemäße Mund- und Zahnpflege- und -reinigungsmittel bevorzugt, die - bezogen auf ihr Gewicht- 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so dass Mittel, die außer Sorbit keine anderen mehrwertigen Alkohole enthalten, besonders bevorzugt sind.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.
Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.
Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.
Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol-Copolymere mit einem mittleren Molgewicht von 1000 bis 5000 oder eine Kombination der genannten Verbindungen in den Zahnpasten verwendet werden.

Ganz besonders gut verträglich mit der erfindungsgemäßen Kombination ist Xanthan. Erfindungsgemäße Mittel, die Xanthan enthalten, ssind außergewöhnlich lagerstabil und weisen einee angenehme Produkthaptik auf. Bevorzugte erfindungsgemäße Mund und Zahnpflege- und - reinigungsmittel sind daher dadurch gekennzeichnet, dass sie zusätzlich 0,1 bis 7,5 Gew.-%, vorzugsweise 0,25 bis 5 Gew.-%, weiter bevorzugt 0,5 bis 2,5 Gew.-% und insbesondere 0,6 bis 1,5 Gew.-% Xanthan enthalten.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nicht- ionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronen- säure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Erfindungsgemäße Mittel können als Zahnpasten oder Zahncremes formuliert werden. Ein weiterer Gegenstand der vorliegenden Erfindung ist die Verwendung von erfindungsgemäßen Mitteln zur Reinigung von Zähnen mittels manuell betätigter oder elektrischer Zahnbürsten. Im Falle elektrischer Zahnbürsten besitzen die erfindungsgemäßen Mittel den weiteren Vorteil, dass sie bereits in geringen Mengen wirksam sind und darüber hinaus die Mechanik des elektrischen Bürstenkopfes nicht beeinträchtigen.

Ein weiterer Gegenstand der vorliegenden Erfindung ist ein Verfahren zum Reinigen von Zähnen, dadurch gekennzeichnet, dass ein erfindungsgemäßes Mittel auf den Bürstenkopf einer elektrischen Zahnbürste aufgetragen und mit der elektrischen Zahnbürste die Zähne geputzt werden.

Ein weiterer Gegenstand der vorliegenden Erfindung ist ein Verfahren zur Zahnreinigung, gekennzeichnet durch die Schritte
a) Bereitstellung einer Zahnbürste, deren Bürstenkopf elektrisch in Bewegung versetzt werden kann;
b) Applikation von 0,5 bis 5 g eines erfindungsgemäßen Mittels auf den Bürstenkopf,
c) 30- bis 300-sekündiges Zähneputzen mit dem erfindungsgemäßen Mittel unter Einsatz des elektrisch in Bewegung versetzten Bürtenkopfes.

Bezüglich bevorzugter Ausführungsformen des erfindungsgemäßen Verfahrens gilt mutatis mutandis das zu den erfindungsgemäßen Mitteln Gesagte.

Durch den Einsatz der erfindungsgemäßen Mittel Polymilchsäure-Partikel kann die Wiederanfärbung von Zähnen effektiv verringert werden. Ein weiterer Gegenstand der vorliegenden Erfindung ist daher ein Verfahren zur Verminderung von Verfärbungen auf der Zahnoberfläche, bei dem ein Mund- und Zahnpflege- und -reinigungsmittel, enthaltend - bezogen auf sein Gewicht -
a) 0,001 bis 5 Gew.-% Copolymer(e), umfassend mindestens eine Struktureinheit der Formel (A1) und mindestens eine Struktureinheit der Formel (A2) 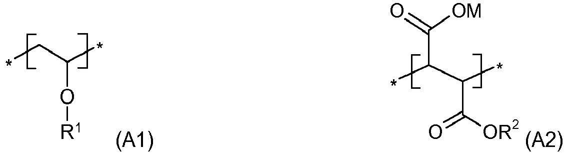 worin
   - R¹: steht für eine (C₁ bis C₁₈)-Alkylgruppe,
   - R²: steht für eine (C₁ bis C₆)-Alkylgruppe,
   - M: steht für ein Wasserstoffatom oder ein Äquivalent eines ein- oder mehrwertigen Kations;
b) 0,001 bis 5 Gew.-% Polymer(e) aus der Gruppe Chitosan und Chitosanderivate;
c) 1 bis 20 Gew.-% Fällungskieselsäure(n) mit einer spezifischen Oberfläche nach ISO 5794-1, Anhang D von ≤ 60 m²/g
auf die Zahnoberflächen appliziert wird.

Bezüglich bevorzugter Ausführungsformen dieses erfindungsgemäßen Verfahrens gilt ebenfalls mutatis mutandis das zu den erfindungsgemäßen Mitteln Gesagte.

### Beispiele:

### Alle Angaben in Gew.-%.

### Beispiel 1 Zahnpastaformulierung

| | **A** | **B** | **C** | **D** | **E** | **F** |
|---|---|---|---|---|---|---|
| Sorbitol, 70%ig | 45 | 50 | 55 | 60 | 65 | 70 |
| Co Polymer* | 0,5 | 0,2 | 0,15 | 0,1 | 0,01 | 0,001 |
| Chitosan | 0,25 | 0,2 | 0,1 | 0,15 | 0,01 | 0,001 |
| Hydrated silica** | 20 | 18 | 17 | 15 | 12,5 | 15 |
| Natriumfluorid | 0,32 | 0,32 | 0,32 | 0,32 | 0,32 | 0,32 |
| Natrium Saccharin | 0,25 | 0,25 | 0,25 | 0,25 | 0,25 | 0,25 |
| Ethanol | - | - | - | 1 | 2 | - |
| Xanthan | 0,2 | 0,1 | 0,3 | 0,2 | 0,2 | 0,1 |
| Natriumlaurylethersulfat | 1,2 | 1,2 | 1,2 | 1,2 | 1,2 | 1,2 |
| Cocamidopropyl Betaine | 0,6 | 0,6 | 0,6 | 0,6 | 0,6 | 0,6 |
| Aroma | 0,1 | 0,1 | 0,1 | 0,1 | 0,1 | 0,1 |
| Wasser | ad 100 | | | | | |

| | | | | | | |
|---|---|---|---|---|---|---|
| * Copolymer aus Maleinsäure und Methylvinylether, MW 1.200.000 Dalton ** Fällungskieselsäure(n) mit einer spezifischen Oberfläche nach ISO 5794-1, Anhang D von ≤ 45 m²/g | | | | | | |

### Beispiel 2 Mundwasserformulierung

| | **A** | **B** | **C** | **D** | **E** | **F** |
|---|---|---|---|---|---|---|
| Sorbitol, 70%ig | 1 | 1,5 | 2 | 2,5 | 3 | 5 |
| Co Polymer* | 0,5 | 0,2 | 0,15 | 0,1 | 0,01 | 0,001 |
| Chitosan | 0,25 | 0,2 | 0,1 | 0,15 | 0,01 | 0,001 |
| Hydrated silica** | 1 | 1,5 | 2 | 2,5 | 1 | 1 |
| Natriumfluorid | 0,11 | 0,11 | 0,11 | 0,11 | 0,11 | 0,11 |
| Natrium Saccharin | 0,03 | 0,05 | 0,1 | 0,05 | 0,05 | 0,1 |
| PEG-60 Hydrogenated Castor Oil | 0,2 | 0,2 | 0,2 | 0,2 | 0,2 | 0,2 |
| Trinatriumcitrat Dihydrat | 0,1 | 0,2 | 0,3 | 0,2 | 0,1 | 0,1 |
| Citronensäure | 0,001 | 0,002 | 0,1 | 0,2 | 0,01 | 0,01 |
| Cetylpyridinium Chloride | 0,01 | 0,1 | 0,05 | 0,05 | 0,1 | 0,01 |
| Aroma | | | | | | |
| Wasser | ad 100 | | | | | |

### Wirkungsnachweis:

Mit den Formulierungen F-E (erfindungsgemäß) und F-V1 (nur Chitosan) bzw. F-V2 (nur Copolymer) wurden Zahnmodelle behandelt, wobei die Zusammensetzungen in identischer Menge und Weise appliziert wurden. Die vorbehandelten Zahnmodelle wurden anschließend bei 20°C 7 Stunden lang in aufgebrühtem Schwarztee gelagert und die Farbveränderung gemessen.

| | **F-E** | **F-V1** | **F-V2** |
|---|---|---|---|
| Sorbitol, 70%ig | 70 | 70 | 70 |
| Co Polymer* | 0,001 | - | 0,002 |
| Chitosan | 0,001 | 0,002 | - |
| Hydrated silica** | 15 | 15 | 15 |
| Natriumfluorid | 0,32 | 0,32 | 0,32 |
| Natrium Saccharin | 0,25 | 0,25 | 0,25 |
| Ethanol | - | - | - |
| Xanthan | 0,1 | 0,1 | 0,1 |
| Natriumlaurylethersulfat | 1,2 | 1,2 | 1,2 |
| Cocamidopropyl Betaine | 0,6 | 0,6 | 0,6 |
| Aroma | 0,1 | 0,1 | 0,1 |
| Wasser | ad 100 | | |

Die folgende Tabelle zeigt die Delta-E-werte der Teeanfärbung nach 7 h auf vorbehandelten Zahnmodellen:

| | **F-E** | **F-V1** | **F-V2** |
|---|---|---|---|
| Delta E | 27,2 | 35,5 | 33,7 |

Die Ergebnisse zeigen deutlich, dass die Wiederanschmutzung mit der erfindungsgemäßen Zusammensetzung am geringsten ausfällt.

## Patentansprüche

1. Mund- und Zahnpflege- und -reinigungsmittel, enthaltend - bezogen auf sein Gewicht -
a) 0,001 bis 5 Gew.-% Copolymer(e), umfassend mindestens eine Struktureinheit der Formel (A1) und mindestens eine Struktureinheit der Formel (A2) 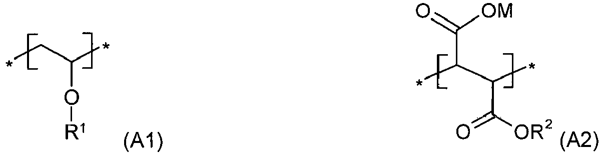 worin
R¹ steht für eine (C₁ bis C₁₈)-Alkylgruppe,
R² steht für eine (C₁ bis C₆)-Alkylgruppe,
M steht für ein Wasserstoffatom oder ein Äquivalent eines ein- oder mehrwertigen Kations;
b) 0,001 bis 5 Gew.-% Polymer(e) aus der Gruppe Chitosan und Chitosanderivate;
c) 1 bis 20 Gew.-% Fällungskieselsäure(n) mit einer spezifischen Oberfläche nach ISO 5794-1, Anhang D von ≤ 60 ₘ²/g.

2. Mund- und Zahnpflege- und -reinigungsmittel nach Anspruch 1, **dadurch gekennzeichnet, dass** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Copolymer(e) aus Maleinsäure und Methylvinylether enthält.

3. Mund- und Zahnpflege- und reinigungsmittel nach einem der Ansprüche 1 oder 2, **dadurch gekennzeichnet, dass** das/die Copolymer(e), umfassend mindestens eine Struktureinheit der Formel (A1) und mindestens eine Struktureinheit der Formel (A2), Molmassen von 500.000 bis 5.000.000 Dalton, vorzugsweise von 600.000 bis 4.000.000 Dalton, weiter bevorzugt von 700.000 bis 3.000.000, noch weiter bevorzugt von 800.000 bis 2.000.000 Dalton und insbesondere von 900.000 bis 1.500.000 Dalton aufweist/aufweisen.

4. Mund- und Zahnpflege- und -reinigungsmittel nach einem der Ansprüche 1 bis 3, **dadurch gekennzeichnet, dass** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% Polymer(e) aus der Gruppe Chitosan und Chitosanderivate enthält.

5. Mund und Zahnpflege- und -reinigungsmittel nach einem der Ansprüche 1 bis 4, **dadurch gekennzeichnet, dass** es 2,5 bis 19,5 Gew.-%, vorzugsweise 5 bis 19 Gew.-%, besonders bevorzugt 7,5 bis 18,5 Gew.-%, weiter bevorzugt 8,0 bis 18 Gew.-% und insbesondere 10,0 bis 17,5 Gew.-% Fällungskieselsäure(n) mit einer spezifischen Oberfläche nach ISO 5794-1, Anhang D von ≤ 55 m²/g enthält.

6. Mund und Zahnpflege- und -reinigungsmittel nach einem der Ansprüche 1 bis 5, **dadurch gekennzeichnet, dass** sämtliche im Mittel enthaltenen Fällungskieselsäure(n) eine spezifische Oberfläche nach ISO 5794-1, Anhang D von ≤ 60 m²/g, vorzugsweise von ≤ 52,5 m²/g, weiter bevorzugt von ≤ 49 m²/g und insbesondere von ≤ 47 m²/g aufweisen.

7. Mund und Zahnpflege- und -reinigungsmittel nach einem der Ansprüche 1 bis 8, **dadurch gekennzeichnet, dass** es 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat enthält.

8. Mund und Zahnpflege- und -reinigungsmittel nach einem der Ansprüche 1 bis 7, **dadurch gekennzeichnet, dass** es - bezogen auf sein Gewicht - weniger als 5 Gew.-%, vorzugsweise weniger als 4 Gew.-%, besonders bevorzugt weniger als 3 Gew.-% und insbesondere weniger als 2 Gew.-% Tensid(e) enthält.

9. Verfahren zur Zahnreinigung, **gekennzeichnet durch** die Schritte
a) Bereitstellung einer Zahnbürste, deren Bürstenkopf elektrisch in Bewegung versetzt werden kann;
b) Applikation von 0,5 bis 5 g eines Mittels nach einem der Ansprüche 1 bis 8 auf den Bürstenkopf,
c) 30- bis 300-sekündiges Zähneputzen mit dem Mittel nach einem der Ansprüche 1 bis 8 unter Einsatz des elektrisch in Bewegung versetzten Bürtenkopfes.

10. Verwendung von Mund- und Zahnpflege- und -reinigungsmitteln, enthaltend - bezogen auf ihr Gewicht -
a) 0,001 bis 5 Gew.-% Copolymer(e), umfassend mindestens eine Struktureinheit der Formel (A1) und mindestens eine Struktureinheit der Formel (A2) 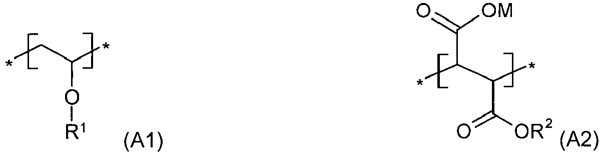 worin
R¹ steht für eine (C₁ bis C₁₈)-Alkylgruppe,
R² steht für eine (C₁ bis C₆)-Alkylgruppe,
M steht für ein Wasserstoffatom oder ein Äquivalent eines ein- oder mehrwertigen Kations;
b) 0,001 bis 5 Gew.-% Polymer(e) aus der Gruppe Chitosan und Chitosanderivate;
c) 1 bis 20 Gew.-% Fällungskieselsäure(n) mit einer spezifischen Oberfläche nach ISO 5794-1, Anhang D von ≤ 60 m²/g
zur Verringerung der Wiederanfärbung von Zähnen.

## Claims

1. An oral and dental care and cleaning agent, containing, based on its weight:
a) from 0.001 to 5 wt.% copolymer(s), comprising at least one structural unit of formula (A1) and at least one structural unit of formula (A2) 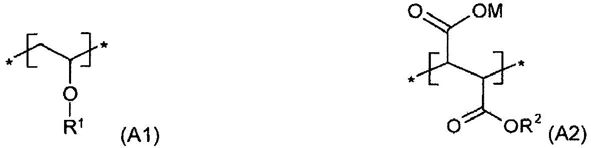 in which
R¹ represents a (C₁ to C₁₈) alkyl group
R² represents a (C₁ to C₆) alkyl group
M represents a hydrogen atom or an equivalent of a monovalent or polyvalent cation;
b) from 0.001 to 5 wt.% polymer(s) from the group comprising chitosan and chitosan derivatives;
c) from 1 to 20 wt.% precipitated silica(s) having a specific surface area according to ISO 5794-1, Annex D, of ≤ 60 m²/g.

2. The oral and dental care and cleaning agent according to claim 1, **characterized in that** said agent contains, based on its weight, from 0.002 to 4 wt.%, preferably from 0.003 to 3 wt.%, particularly preferably from 0.004 to 2 wt.%, exceptionally preferably from 0.005 to 1 wt.%, and in particular from 0.01 to 0.5 wt.% copolymer(s) from maleic acid and methyl vinyl ether.

3. The oral and dental care and cleaning agent according to one of claims 1 or 2, **characterized in that** the copolymer(s), which comprise(s) at least one structural unit of formula (A1) and at least one structural unit of formula (A2), has/have molecular masses of from 500,000 to 5,000,000 daltons, preferably from 600,000 to 4,000,000 daltons, more preferably from 700,000 to 3,000,000 daltons, even more preferably from 800,000 to 2,000,000 daltons, and in particular from 900,000 to 1,500,000 daltons.

4. The oral and dental care and cleaning agent according to one of claims 1 to 3, **characterized in that** said agent contains, based on its weight, from 0.002 to 4 wt.%, preferably from 0.003 to 3 wt.%, particularly preferably from 0.004 to 2 wt.%, exceptionally preferably from 0.005 to 1 wt.%, and in particular from 0.01 to 0.5 wt.% polymer(s) from the group comprising chitosan and chitosan derivatives.

5. The oral and dental care and cleaning agent according to one of claims 1 to 4, **characterized in that** said agent contains from 2.5 to 19.5 wt.%, preferably from 5 to 19 wt.%, particularly preferably from 7.5 to 18.5 wt.%, more preferably from 8.0 to 18 wt.%, and in particular from 10.0 to 17.5 wt.% precipitated silica(s) having a specific surface area according to ISO 5794-1, Annex D, of ≤ 55 m²/g.

6. The oral and dental care and cleaning agent according to one of claims 1 to 5, **characterized in that** all the precipitated silica(s) contained in the agent has/have a specific surface area according to ISO 5794-1, Annex D, of ≤ 60 m²/g, preferably of ≤ 52.5 m²/g, more preferably ≤ 49 m²/g, and in particular ≤ 47 m²/g.

7. The oral and dental care and cleaning agent according to one of claims 1 to 8, **characterized in that** said agent contains from 0.01 to 2.5 wt.%, preferably from 0.05 to 2.0 wt.%, particularly preferably from 0.1 to 1.0 wt.%, more preferably from 0.11 to 0.75 wt.%, and in particular from 0.12 to 0.5 wt.% calcium glycerophosphate.

8. The oral and dental care and cleaning agent according to one of claims 1 to 7, **characterized in that** said agent contains, based on its weight, less than 5 wt.%, preferably less than 4 wt.%; particularly preferably less than 3 wt.%, and in particular less than 2 wt.% surfactant(s).

9. A method for cleaning teeth, **characterized by** the steps of:
a) providing a toothbrush, the brush head of which can be set in motion electrically;
b) applying from 0.5 to 5 g of an agent according to one of claims 1 to 8 to the brush head;
c) brushing the teeth with the agent according to one of claims 1 to 8 for from 30 to 300 seconds using the brush head that can be set in motion electrically.

10. The use of oral and dental care and cleaning agents containing, based on the weight thereof:
a) from 0.001 to 5 wt.% copolymer(s), comprising at least one structural unit of formula (A1) and at least one structural unit of formula (A2) 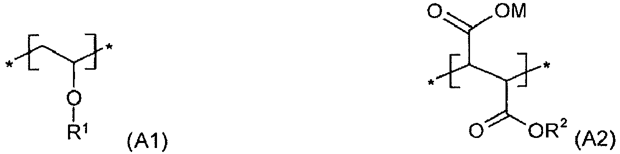 in which
R¹ represents a (C₁ to C₁₈) alkyl group
R² represents a (C₁ to C₆) alkyl group
M represents a hydrogen atom or an equivalent of a monovalent or polyvalent cation;
b) from 0.001 to 5 wt.% polymer(s) from the group comprising chitosan and chitosan derivatives;
c) from 1 to 20 wt.% precipitated silica(s) having a specific surface area according to ISO 5794-1, Annex D, of ≤ 60 m²/g
for reducing restaining of teeth.

## Revendications

1. Agent de nettoyage et de soin bucco-dentaire, contenant sur la base de son poids
a. de 0,001 à 5% en poids de copolymère(s) comportant au moins un motif structural de la formule (A1) et au moins un motif structural de la formule (A2) 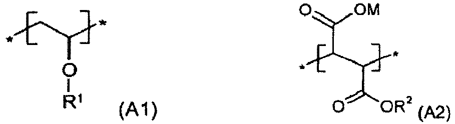 où
R¹ représente un groupe alkyle en C₁ à C₁₈,
R² représente un groupe alkyle en C₁ à C₆,
M représente un hydrogène ou un équivalent d'un cation monovalent ou multivalent ;
b. de 0,001 à 5% en poids de polymère(s) du groupe comportant le chitosane et des dérivés du chitosane ;
c. de 1 à 20% en poids d'acide(s) silique(s) précipité(s) ayant une surface spécifique, selon la norme ISO 5794-1, annexe D, ≤ 60 m²/g.

2. Agent de nettoyage et de soin bucco-dentaire selon la revendication 1, **caractérisé en ce qu'**il contient, sur la base de son poids, de 0,002 à 4% en poids, avantageusement de 0,003 à 3% en poids, de façon particulièrement préférée de 0,004 à 2% en poids, de façon extraordinairement préférée de 0,005 à 1 % en poids et en particulier de 0,01 à 0,5% de copolymère(s) d'acide maléique et d'éther méthylvinylique.

3. Agent de nettoyage et de soin bucco-dentaire selon l'une des revendications 1 ou 2, **caractérisé en ce que** le ou les copolymère(s), comportant au moins un motif structural de la formule (A1) et au moins un motif structural de la formule (A2), ont des poids moléculaires de 500 000 à 5 000 000 daltons, avantageusement de 600 000 à 4 000 000 daltons, plus préférablement de 700 000 à 3 000 000, encore plus préférablement de 800 000 à 2 000 000 daltons et en particulier de 900 000 à 1 500 000 daltons.

4. Agent de nettoyage et de soin bucco-dentaire selon l'une des revendications 1 à 3, **caractérisé en ce qu'**il contient, sur la base de son poids, de 0,002 à 4% en poids, de préférence de 0,003 à 3% en poids, de façon particulièrement préférée de 0,004 à 2% en poids, de façon extraordinairement préférée de 0,005 à 1% en poids et en particulier de 0,01 à 0,5% en poids de polymère(s) du groupe du chitosane et des dérivés du chitosane.

5. Agent de nettoyage et de soin bucco-dentaire selon l'une des revendications 1 à 4, **caractérisé en ce qu'**il contient de 2,5 à 19,5% en poids, de préférence de 5 à 19% en poids, de façon particulièrement préférée de 7,5 à 18,5% en poids, plus préférablement de 8,0 à 18% en poids et en particulier de 10,0 à 17,5% en poids d'acide(s) silique(s) précipité(s) ayant une surface spécifique, selon ISO 5794-1, annexe D, ≤ 55 m²/g.

6. Agent de nettoyage et de soin bucco-dentaire selon l'une des revendications 1 à 5, **caractérisé en ce que** tous les acides siliques précipités contenus dans l'agent ont une surface spécifique, selon la norme ISO 5794-1, annexe D, de ≤ 60 m²/g, de préférence ≤ 52,5 m²/g, plus préférablement ≤ 49 m²/g et en particulier ≤ 47 m²/g.

7. Agent de nettoyage et de soin bucco-dentaire selon l'une des revendications 1 à 8, **caractérisé en ce qu'**il contient de 0,01 à 2,5% en poids, de préférence de 0,05 à 2,0% en poids, de manière particulièrement préférée de 0,1 à 1,0% en poids, plus préférablement de 0,11 à 0,75% en poids et en particulier de 0,12 à 0,5% en poids de glycérophosphate de calcium.

8. Agent de nettoyage et de soin bucco-dentaire selon l'une des revendications 1 à 7, **caractérisé en ce qu'**il contient, sur la base de son poids, moins de 5% en poids, de préférence moins de 4% en poids, plus préférablement moins de 3% en poids et en particulier moins de 2% en poids de tensioactif(s).

9. Procédé de nettoyage dentaire, **caractérisé par** les étapes consistant à
a. produire une brosse à dent dont la tête peut être mise en mouvement électriquement ;
b. appliquer de 0,5 à 5 g d'un agent selon l'une des revendications 1 à 8 sur la tête de la brosse,
c. brosser les dents pendant 30 à 300 secondes avec l'agent selon l'une des revendications 1 à 8 au moyen de a tête de brosse mise en mouvement électriquement.

10. Utilisation d'agents de nettoyage et de soin bucco-dentaire, contenant sur la base de leur poids
a. de 0,001 à 5% en poids de copolymère(s) comportant au moins un motif structural de la formule (A1) et au moins un motif structural de la formule (A2) 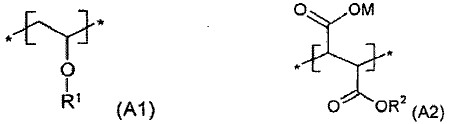 où
R¹ représente un groupe alkyle en C₁ à C₁₈,
R² représente un groupe alkyle en C₁ à C₆,
M représente un hydrogène ou un équivalent d'un cation monovalent ou multivalent ;
b. de 0,001 à 5% en poids de polymère(s) du groupe comportant le chitosane et des dérivés du chitosane ;
c. de 1 à 20% en poids d'acide(s) silique(s) précipité(s) ayant une surface spécifique, selon la norme ISO 5794-1, annexe D, ≤ 60 m²/g
pour réduire la recoloration des dents.
